# EUROPEAN PATENT SPECIFICATION

(11) **EP 0 423 991 B1**
(45) Date of publication and mention of the grant of the patent: **14.06.1995**
(21) Application number: 90310926.2
(22) Date of filing: 04.10.1990
(51) Int. Cl.: C07C 65/03

(54) **Process for the preparation of 2-trifluoromethyl-4-hydroxybenzoic acid**
Verfahren zur Herstellung von 2-Trifluormethyl-4-hydroxybenzoesäure
Procédé de préparation d'acide 2-trifluorméthyl-4- hydroxybenzoique

(30) Priority: 20.10.1989 JP 273437/89
(43) Date of publication of application: 24.04.1991
(73) Proprietor: Showa Shell Sekiyu Kabushiki Kaisha, Tokyo (JP)
(72) Inventor: Suzuki, Yoshiichi, c/o Showa Shell, Chiyoda-ku, Tokyo 100 (JP)
(74) Representative: Connor, Terence Kevin

(56) References cited:
- EP-A- 0 134 959
- US-A- 3 790 528
- JOURNAL OF THE AMERICAN CHEMICAL SOCIETY, vol. 76, no. 4, 6 March 1954, Gaston, PA, US; M. HAUPTSCHEIN et al, "Trifluoromethyl derivatives of hydroxybenzoic acids and related compounds", pp. 1051-1054

## Description

The present invention relates to a novel process for preparing 2-tri-fluoromethyl-4-hydroxybenzoic acid.

The known process of preparing 2-tri-fluoromethyl-4-hydroxybenzoic acid is disclosed in J. Am. Chem. Soc. vol. 76, no. 4. March 1954,

The present invention relates to a novel process for preparing 2-tri-fluoromethyl-4-hydroxybenzoic acid represented by the following formula: 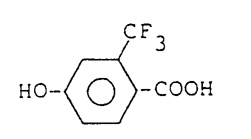
(hereinafter referred to as the present compound).

The present compound is prepared through the following synthesis route. 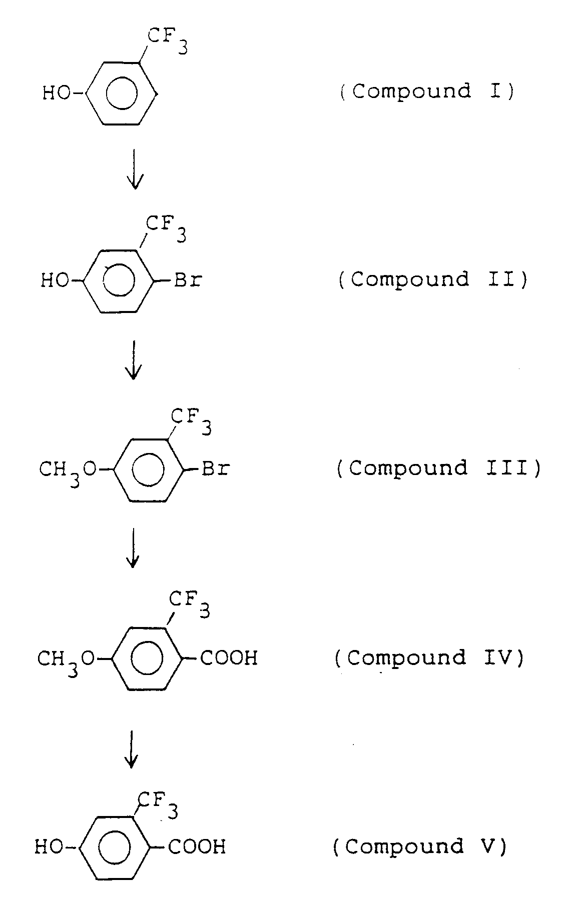
The present compound is used as a raw material for condensation polymers such as polyesters; organic functional materials; liquid crystals; physiologically active substances; and intermediates for pharmaceutical and agricultural chemicals.

### Example

### 1. Preparation of 3-trifluoromethyl-4-bromophenol (Compound II):

3-Trifluoromethylphenol (compound I) (5.0 g) was dissolved in carbon disulfide (3.0 ml) and then to the solution was added a solution of bromine (5.0 g) in carbon disulfide (2.0 ml) over a period of about 15 minutes at 25 °C or less, followed by allowing the reaction to proceed for 2 hours at 25 °C. The resulting reaction mixture was added to water (10 ml) and then subjected to repeated extraction with methylene chloride. The methylene chloride layer was washed with aqueous sodium hydrogen-carbonate solution and then with water and dehydration-dried by adding anhydrous sodium sulfate. The solvent was removed by distillation under reduced pressure to obtain 3-trifluoromethyl-4-bromophenol (compound II, 4.4 g).

### 2. Preparation of 3-trifluoromethyl-4-bromo-1-methoxybenzene (compound III):

A solution prepared by dissolving 3-trifluoromethyl-4-bromo-1-phenol (4.4 g) in dehydrated dimethylformamide (20 ml) was added dropwise to a solution prepared by suspending 60% sodium hydride (0.86 g) in dehydrated dimethylformamide (10 ml) over a period of about 30 minutes at 25 °C or lower. Then, the mixture was stirred at room temperature for 30 minutes and then, methyl iodide (3.1 g) was slowly added thereto at 30 °C or lower and reaction was allowed to proceed at room temperature for 1 hour. Unreacted 60% sodium hydride was decomposed by conventional method and the reaction mixture was added to water (60 ml) and extracted with ethyl acetate and ethyl acetate layer was sufficiently washed with water and dehydrated by adding anhydrous sodium sulfate thereto. The solvent was removed by distillation under reduced pressure and the residue was purified by distillation to obtain 3-trifluoromethyl-4-bromo-1-methoxybenzene (compound III, 4.4 g).

### 3. Preparation of 2-trifluoromethyl-4-methoxybenzoic acid (compound IV):

Dry tetrahydrofuran (10 ml) was added to 3-trifluoromethyl-4-bromo-1-methoxybenzene (compound III) (4.4 g) and metallic magnesium (0.41 g) and thereto was further added a catalytic amount of iodine, followed by refluxing for 1.5 hours with stirring. After the temperature was returned to room temperature, to the mixture was slowly added dropwise a solution prepared by adding dry ice (30 g) to dry tetrahydrofuran (10 ml) at -40 °C or lower. The temperature was returned to room temperature and then the reaction was allowed to proceed for further 2 hours. Then, the reaction mixture was added to 1N hydrochloric acid (50 ml) to stop the reaction and repeatedly extracted with ethyl acetate. The ethyl acetate layer was sufficiently washed with water and dried by adding anhydrous sodium sulfate. The solvent was removed by distillation under reduced pressure and then, the resulting solid was recrystallized from methylene chloride to obtain 2-trifluoromethyl-4-methoxybenzoic acid (compound IV, 1.5 g).

### 4. Preparation of 2-trifluoromethyl-4-hydroxybenzoic acid (compound V):

A solution prepared by dissolving 2-trifluromethyl-4-methoxybenzoic acid (compound IV) (1.0 g) in methylene chloride (10 ml) was slowly added dropwise to a solution prepared by dissolving boron tribromide (2.5 g) in methylene chloride (10 ml) at 30 °C or lower with stirring, followed by stirring for further 20 minutes.

This solution was added to ice water (50 g) and then repeatedly extracted with ethyl ether. The ethyl ether layer was washed with water and dried by adding anhydrous sodium sulfate and the solvent was distilled off to obtain a crude product. This was recrystallized from toluene to obtain 2-trifluoromethyl-4-hydroxybenzoic acid (compound V, 0.5 g).

## Claims

1. A process for preparing 2-tri-fluoromethyl-4-hydroxybenoic acid represented by the formula: 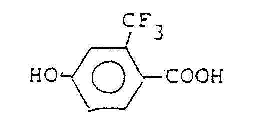 which comprises allowing the compound represented by the formula: 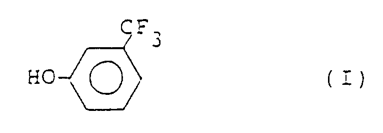 to react with bromine to prepare the compound represented by the formula: 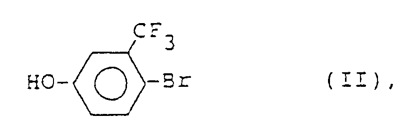 allowing the compound (II) to react with methyl iodide to prepare the compound represented by the formula: 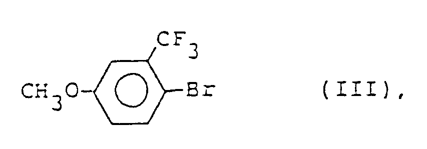 allowing the compound (III) to react with magnesium and CO₂ to prepare the compound represented by the formula: 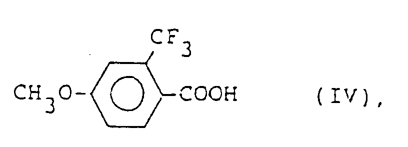 and allowing the compound (IV) to react with boron tribromide.

## Patentansprüche

1. Ein Verfahren zur Herstellung von 2-Trifluormethyl-4-hydroxybenzoesäure, dargestellt durch die Formel 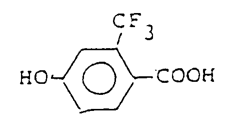 welches die Umsetzung der Verbindung, dargestellt durch die Formel 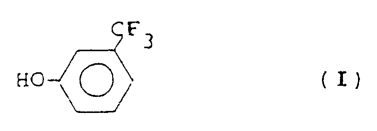 mit Brom zwecks Herstellung der Verbindung , dargestellt durch die Formel 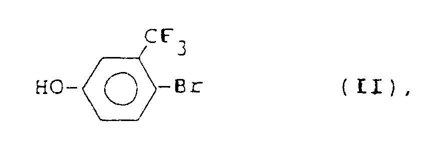 die Umsetzung der Verbindung (II) mit Methyljodid zwecks Herstellung der Verbindung, dargestellt durch die Formel 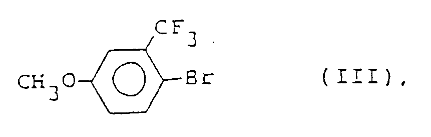 die Umsetzung der Verbindung (III) mit Magnesium und CO₂ zwecks Herstellung der Verbindung, dargestellt durch die Formel 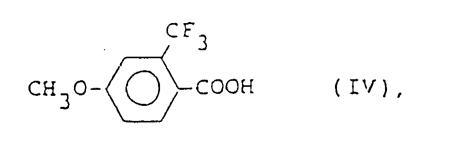 und die Umsetzung der Verbindung (IV) mit Bortribromid umfaßt.

## Revendications

1. Procédé de préparation de l'acide 2-trifluorométhyl-4-hydroxybenzoique représenté par la formule: 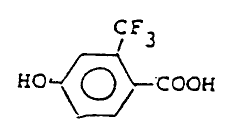 qui consiste à mettre le composé représenté par la formule : 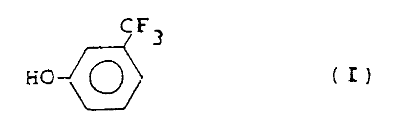 à réagir sur du brome pour préparer le composé représenté par la formule : 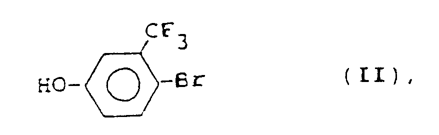 à mettre le composé (II) à réagir sur de l'iodure de méthyle pour préparer le composé représenté par la formule : 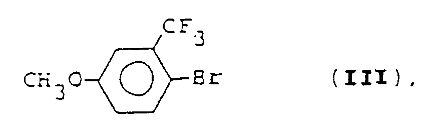 à mettre le composé (III) à réagir sur du magnésium et du CO₂ pour préparer le composé représenté par la formule : 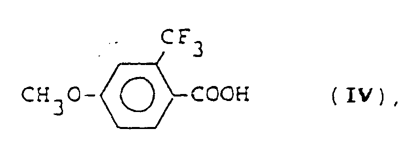 et à mettre le composé (IV) à réagir sur du tribromure de bore.
